# EUROPÄISCHE PATENTSCHRIFT

(11) **EP 0 508 264 B1**
(45) Veröffentlichungstag und Bekanntmachung des Hinweises auf die Patenterteilung: **26.04.1995**
(21) Anmeldenummer: 92105452.4
(22) Anmeldetag: 30.03.1992
(51) Int. Cl.: C07C 303/32, C07C 51/353, C07C 67/343, C07C 309/29, C07C 309/32, C07C 63/66, C07C 69/76, C07C 69/618

(54) **Verfahren zur Arylierung von Olefinen**
Process for the arylation of olefines
Procédé pour l'arylation d'oléfines

(30) Priorität: 10.04.1991 DE 4111653; 10.04.1991 DE 4111620; 10.04.1991 DE 4111657
(43) Veröffentlichungstag der Anmeldung: 14.10.1992
(73) Patentinhaber: BAYER AG, 51368 Leverkusen (DE)
(72) Erfinder: Bader, Axel, Dr., W-5090 Leverkusen 1 (DE); Arlt, Dieter, Prof. Dr., W-5000 Köln 80 (DE); Fiedel, Dietmar, Dr., W-5068 Odenthal-Voiswinkel (DE); Meier, Stefan, Dr., W-5090 Leverkusen 3 (DE)

(56) Entgegenhaltungen:
- EP-A- 0 405 389
- DE-A- 2 325 302
- BULLETIN OF THE CHEMICAL SOCIETY OF JAPAN. Bd. 52, Nr. 9, September 1979, TOKYOJP Seiten 2609 - 2610; K. KIKUKAWA ET AL.: 'Reaction of Diazonium Salts with Transition Metalls. II. Palladium-catalyzed Arylation of Ethylene with Arenediazonium Salts'
- TETRAHEDRON, (INCL. TETRAHEDRON REPORTS) Bd. 37, Nr. 1, 1981, OXFORD GB Seiten 31 - 36; K. KIKUKAWA ET AL.: 'Reaction of Diazonium Salts with Transition Metalls - III. Palladium(0)-Catalyzed Arylation of Unsaturated Compounds with Arenediazonium Salts'

## Beschreibung

Die vorliegende Erfindung betrifft ein Verfahren zur Herstellung von Arylolefinen durch Umsetzung von Aryldiazoniumsalzen und Olefinen in Gegenwart eines Palladium-Katalysators.

In Bull. Chem. Soc. Jap. 52 (9), 1979, S. 2609/2610 und in Tetrahedron 37 (1), S. 31f. werden Palladium(0)-katalysierte Arylierungen von ungesättigten Verbindungen mit Aryldiazoniumsalzen beschrieben. Die beschriebenen Verfahren werden entweder mit Aryldiazonium-tetrafluoboraten oder -chloriden ausgeführt. Während die Herstellung der Tetrafluoborate das Arbeiten mit der unangenehmen Fluorborsäure erfordert, ergeben die Chloride keine optimalen Ausbeuten.

Gegenstand der Erfindung ist ein Verfahren zur Herstellung von Verbindungen der Formel (I) 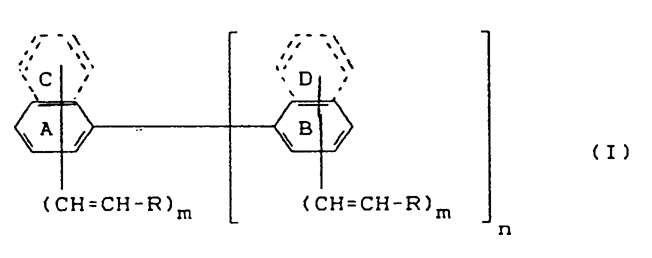
worin
- R: für H, CN, COOH, COO-C₁-C₁₂-Alkyl, SO₃M, C₁-C₁₂-Alkyl, gegebenenfalls substituiertes C₆-C₁₀-Aryl, gegebenenfalls substituiertes Hetaryl mit 3 bis 5 Kohlenstoffatomen und 1 bis 3 Heteroatomen aus der Reihe Sauerstoff, Schwefel, Stickstoff steht,
- n: für 0 oder 1 steht,
wobei für den Fall, daß n für Null steht, die freie Valenz an Ring A durch Wasserstoff abgesättigt ist,
- m: für 1 oder 2 steht
und
die Ringe A, B, C, D und die genannten Aryl- oder Hetarylreste weiterhin durch einen oder mehrere Substituenten aus der Reihe Fluor, Chlor, Brom, Iod, C₁-C₁₂-Alkyl, C₁-C₁₂-Alkoxy, CF₃, OCF₃, CN, NO₂, COOM, COO-C₁-C₁₂-Alkyl, SO₃M, CO-C₁-C₁₂-Alkyl, CO-C₆-C₁₀-Aryl, C₆-C₁₀-Aryl, Hetaryl, gegebenenfalls durch C₁-C₁₂-Alkyl substituiertes Amino, substituiert sein können und
- M: ein Proton, ein Alkalimetallion, ein Erdalkalimetallion oder Ammonium bedeutet,
durch Umsetzung einer Verbindung der Formel (II) 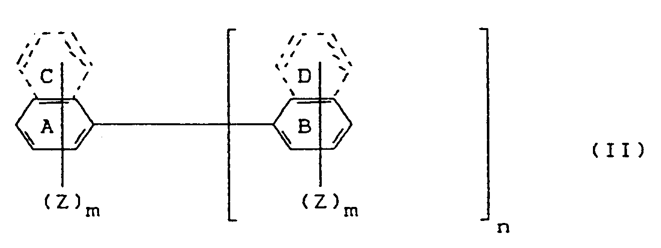
worin
- Z: für N₂^{⊕}G steht,
- G: für Sulfat oder Hydrogensulfat steht und
m und n die oben genannte Bedeutung haben,
wobei für den Fall, daß n für Null steht, die freie Valenz an Ring A durch Wasserstoff abgesättigt ist,
und die Ringe A, B, C und D gegebenenfalls durch einen oder mehrere der oben für diese Ringe genannten Substituenten substituiert sein können,
mit einer Verbindung der Formel (III)

R-CH=CH₂ (III)

worin
- R: die oben genannte Bedeutung hat,
in Gegenwart eines anorganischen oder organischen Palladiumsalzes oder deren Mischung als Katalysator, wobei die Reaktion in Wasser, Alkohol oder einer Mischung daraus durchgeführt wird.

Insbesondere können die Arylreste in der Definition von R für die Ringe A und B oder die anellierten Ringsysteme AC bzw. BD stehen.

Tragen die Ringe A, B, C oder D negativ geladene Substituenten wie z.B. SO₃⁻, dann können die Verbindungen der Formel (II) auch als innere Salze vorliegen.

Als Kationen M geeignete Alkaliionen sind insbesondere Natrium- und Kaliumionen.

Als Beispiele für Verbindungen der Formel (II) seien genannt:
Die isomeren Diazobenzolsulfonsäuren wie 2-, 3- oder 4-Diazobenzolsulfonsäure, die isomeren Bis-(diazo)-benzolsulfonsäuren wie 2,4-Bis-(diazo)-benzolsulfonsäure oder 2,5-Bis-(diazo)-benzolsulfonsäure, die isomeren Diazobenzoldisulfonsäuren wie Diazobenzol-2,4-disulfonsäure, Diazobenzol-3,5-disulfonsäure und Diazobenzol-2,5-disulfonsäure, die isomeren Bis-(diazo)-benzoldisulfonsäuren wie 1,4-Bis-(diazo)-benzol-2,6-disulfonsäure oder 1,3-Bis-(diazo)-benzol-4,6-disulfonsäure, weiterhin 2-, 3- oder 4-Diazobenzoesäure, 2-, 3- oder 4-Diazobenzoesäure-C₁-C₁₂-alkylester, wie 2-, 3- oder 4-Diazobenzoesäuremethylester, 2-, 3- oder 4-Diazobenzoesäureethylester, 2-, 3- oder 4-Diazobenzoesäure-n-propylester, 2-, 3- oder 4-Diazobenzoesäure-i-propylester, 2-, 3- oder 4-Diazobenzoesäure-n-butylester, 2-, 3- oder 4-Diazobenzoesäure-C₆-C₁₀-arylester wie 2-, 3- oder 4-Diazobenzoesäurephenylester, 3- oder 4-Diazobenzol-1,2-dicarbonsäure, 3- oder 4-Diazobenzol-1,2-dicarbonsäure-di-C₁-C₁₂-alkylester wie 3- oder 4-Diazobenzol-1,2-dicarbonsäuredimethylester oder 3- oder 4-Diazobenzol-1,2-dicarbonsäurediethylester, 3- oder 4-Diazobenzol-1,2-dicarbonsäureanhydrid, 2-, 3- oder 4-Diazobenzonitril, 3- oder 4-Diazophthalodinitril oder Diazo-C₁-C₁₂-alkoxybenzole wie 2-, 3- oder 4-Diazomethoxybenzol, 2-, 3- oder 4-Diazoethoxybenzol, 2-, 3- oder 4-Diazo-tert.-butoxybenzol, 2-, 3- oder 4-Diazophenyl-C₆-C₁₀-arylether, 2-, 3- oder 4-Diazonitrobenzol, 2-, 3-oder 4-Diazofluorbenzol, 2-, 3- oder 4-Diazochlorbenzol, 2-, 3- oder 4-Diazobrombenzol, 2-, 3- oder 4-Diazoiodbenzol, die isomeren Diazofluorchlorbenzole wie 3-Diazo-2-fluorchlorbenzol, die isomeren Diazobromchlorbenzole, die isomeren Diazofluorbrombenzole, die isomeren Diazodifluorbenzole, die isomeren Diazodichlorbenzole, die isomeren Diazodibrombenzole, die isomeren Diazodiiodbenzole, 2-, 3- oder 4-Diazo(trifluormethylbenzol), (2-, 3- oder 4-Diazophenyl)-C₁-C₁₂-alkylketone wie 2-, 3-oder 4-Diazoacetophenon, (2-, 3- oder 4-Diazophenyl)-C₆-C₁₀-Arylketone wie 2-, 3- oder 4-Diazobenzophenon, 2-, 3- oder 4-Diazo-C₁-C₁₂-Akylbenzole wie 2-, 3- oder 4-Diazotoluol, die isomeren 2-, 3- oder 4-Diazodi-C₁-C₁₂-alkylbenzole wie 3- oder 4-Diazo-o-xylol, die isomeren Bis-(diazo)-C₁-C₁₂-alkylbenzole, wie 2,3-Bis-(diazo)-toluol, 2,4-Bis-(diazo)-toluol, 2,5-Bis-(diazo)-toluol oder 2,6-Bis-(diazo)-toluol, ferner Bis-(diazo)-di-C₁-C₁₂-alkylbenzole, 2-, 3- oder 4-Diazo-C₆-C₁₀-aryl-benzole wie 2-, 3- oder 4-Diazobiphenyl, 2-, 3- oder 4-Diazoaminobenzol, 2-, 3- oder 4-Diazophenol. Weiter seien genannt die isomeren Diazonaphthalinsulfonsäuren wie 8-Diazo-2-naphthalinsulfonsäure, 8-Diazo-1-naphthalinsulfonsäure, 7-Diazo-1-naphthalinsulfonsäure, 6-Diazo-2-naphthalinsulfonsäure, 5-Diazo-2-naphthalinsulfonsäure, 5-Diazo-1-naphthalinsulfonsäure, 4-Diazo-2-naphthalinsulfonsäure, 2-Diazonaphthalin-1-sulfonsäure, 1-Diazo-2-naphthalinsulfonsäure, die isomeren Diazonaphthalindisulfonsäuren wie 7-Diazo-1,3-naphthalindisulfonsäure, 3-Diazo-2,6-naphthalindisulfonsäure, 3-Diazo-2,7-naphthalindisulfonsäure, 4-Diazo-1,3-naphthalindisulfonsäure, 4-Diazo-1,5-naphthalindisulfonsäure, 4-Diazo-1,6-naphthalindisulfonsäure, 4-Diazo-1,7-naphthalindisulfonsäure, 4-Diazo-2,6-naphthalindisulfonsäure, 6-Diazo-1,3-naphthalindisulfonsäure, 8-Diazo-1,3-naphthalin-disulfonsäure, 3-Diazo-1,5-naphthalindisulfonsäure, 4-Diazo-2,7-naphthalindisulfonsäure, 5-Diazo-1,3-naphthalindisulfonsäure, die isomeren Bis-(diazo)-naphthalinsulfonsäuren und Bis-(diazo)-naphthalindisulfonsäuren wie 3,4-Bis-(diazo)-1-naphthalinsulfonsäure, 4,5-Bis-(diazo)-1-naphthalinsulfonsäure, 3,8-Bis-(diazo)-1,5-naphthalindisulfonsäure, 4,8-Bis-(diazo)-2,6-naphthalin-disulfonsäure, 5,6-Bis-(diazo)-1,3-naphthalindisulfonsäure, 4,5-Bis-(diazo)-2,7-naphthalin disulfonsäure, ferner Bis-(diazo)-biphenyl-mono- und disulfonsäuren wie 4,4'-Bis-(diazo)-biphenyl-3-sulfonsäure und 4,4'-Bis-(diazo)-3,3'-disulfonsäure.

Als Beispiele für Verbindungen der Formel (III) seien genannt Ethylen, Propylen, Butylen, gegebenenfalls substituierte Vinyl-C₆-C₁₀-aromaten wie Styrol oder die isomeren Vinylnaphthaline, 2-, 3- oder 4-Fluorstyrol, 2-, 3- oder 4-Chlorstyrol, 2-, 3- oder 4-Bromstyrol, 2-, 3- oder 4-Iodstyrol, 2-, 3- oder 4-Cyanostyrol, 2-, 3- oder 4-Nitrostyrol, 2-, 3- oder 4-Styrolcarbonsäure, 2-, 3- oder 4-Styrolcarbonsäure-C₁-C₁₂-alkylester wie 2-, 3- oder 4-Styrolcarbonsäuremethylester, 2-, 3- oder 4-Styrolcarbonsäure-C₆-C₁₂-Arylester wie 2-, 3- oder 4-Styrolcarbonsäurephenylester, 2-, 3- oder 4-Styrolsulfonsäure bzw. deren Salze, 3- oder 4-Vinylphthalsäure, 3- oder 4-Vinylphthalsäuredi-C₁-C₁₂-alkylester wie 3- oder 4-Vinylphthalsäuredimethylester, 3- oder 4-Vinylphthalsäuredi-C₆-C₁₀-arylester wie 3-oder 4-Vinylphthalsäurediphenylester, 3- oder 4-Vinylphthalsäureanhydrid, Vinylhetaryle wie N-Vinylimidazol oder 2- oder 4-Vinylpyridin, ferner Acrylnitril, Acrylsäure, Acrylsäure-C₁-C₁₂-alkylester wie Acrylsäuremethylester, Acrylsäureethylester, Acrylsäure-n-propylester, Acrylsäure-2-ethyl-hexylester, Vinylsulfonsäure bzw. deren Salze.

Geeignete organische bzw. anorganische Palladiumsalze sind beispielsweise das Chlorid, das Bromid, das Iodid, das Nitrat, das Sulfat, das Acetat oder das Propionat; bevorzugt sind die genannten Halogenide und das Acetat.

Die genannten Stoffe können allein oder in beliebigen Gemischen als Katalysatoren eingesetzt werden.

In bestimmten Fällen kann es von Vorteil sein, bei der Durchführung des erfindungsgemäßen Verfahrens Verbindungen zuzusetzen, die mit Palladium oder Palladiumsalzen Komplexe bilden. Geeignete Komplexbildner sind z.B. gegebenenfalls durch Sulfogruppen substituierte Arylnitrile wie Benzonitril oder C₁-C₄-Alkylnitrile wie Acetonitril oder Phosphite wie z.B. Triphenylphosphit. Bevorzugt sind als Komplexbildner Phosphane wie Triarylphosphane der Formel (IV) 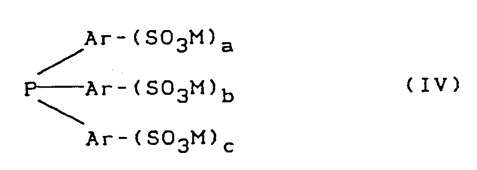
worin
- Ar: für gegebenenfalls substituiertes C₆-C₁₀-Aryl, vorzugsweise für gegebenenfalls durch Methyl substituiertes Phenyl steht,
- a, b und c: unabhängig voneinander für 0, 1 oder 2 stehen, wobei die Summe von a, b und c vorzugsweise maximal 3 beträgt, und
- M: die oben angegebene Bedeutung hat,
sowie Bis(diarylphosphan)-alkane der Formel (V) 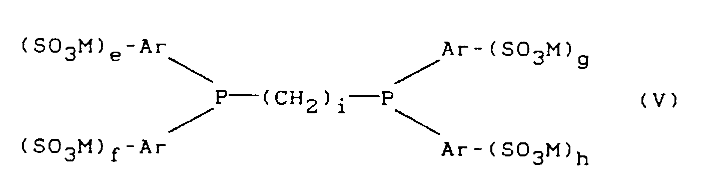
worin
- i: für 1, 2, 3 oder 4 steht,
- Ar: für gegebenenfalls substituiertes C₆-C₁₀-Aryl, vorzugsweise für gegebenenfalls durch Methyl substituiertes Phenyl steht,
- e, f, g und h: unabhängig voneinander für 0 oder 1 stehen, wobei die Summe von e, f, g und h vorzugsweise maximal 4 beträgt, und
- M: die oben angegebene Bedeutung hat.

Beispiele für Triarylphosphane der Formel (IV) sind Tri-o-tolylphosphan, Triphenylphosphan, Triphenylphosphan-tri-(natriumsulfonat), Triphenylphosphan-di-(natriumsulfonat), Triphenylphosphan-mono-(natriumsulfonat).

Beispiele für Bis(diarylphosphan)-alkane der Formel (V) sind Bis(diphenylphosphan)-methan, -ethan, -propan, - butan, Bis-(diphenylphosphan)methan-mono-, di-, tri-, oder tetra-(natriumsulfonat), Bis-(diphenylphosphan)-ethan-mono-, di-, tri- oder tetra-(natriumsulfonat), Bis-(diphenylphosphan)propan-mono-, di-, tri- oder tetra-(natriumsulfonat).

Ebenfalls als Komplexbildner geeignet sind gegebenenfalls durch Sulfogruppen substituierte Trihetarylphosphane, wie z.B. Trifurylphosphan. Weiterhin geeignet ist gegebenenfalls durch Sulfogruppen substituiertes Dibenzylidenaceton.

Wenn das erfindungsgemäße Verfahren mit Wasser als Lösungsmittel durchgeführt wird, sind als Komplexbildner im allgemeinen solche bevorzugt, die hydrophile Reste wie z.B. COOM, OH, NH₂ oder SO₃M enthalten, wobei M die oben angegebene Bedeutung hat, insbesondere solche der Formel (IV), die 1 bis 3 Sulfogruppen enthalten, und/oder solche der Formel (V), die 1 bis 4 Sulfogruppen enthalten.

Wird das erfindungsgemäße Verfahren in einem organischen Lösungsmittel durchgeführt, so sind im allgemeinen sulfogruppenfreie Komplexbildner bevorzugt, insbesondere solche der Formel (IV), worin die Summe von a, b und c Null ist und/oder der Formel (V), worin die Summe von e, f, g und h Null ist.

Die Komplexe werden vorzugsweise in situ erzeugt; es können aber auch vorgebildete Komplexe der genannten Komplexbildner mit Palladium eingesetzt werden. Beispiele für solche vorgebildeten Komplexe sind Tetrakis-(triphenylphosphan)palladium, Bis(dibenzylidenaceton)palladium, Tris-(dibenzylidenaceton)-dipalladium, Bis(triphenylphosphan)palladiumdichlorid, Bis(benzonitril)palladiumdichlorid oder auch Bis(acetonitril)palladiumdichlorid.

Es können auch Mischungen der genannten Komplexbildner eingesetzt werden. Die genannten Komplexbildner können im Überschuß eingesetzt werden; im allgemeinen verwendet man pro mol Palladium 2 bis 40 mol der genannten Komplexbildner. Die Katalysatoren werden im allgemeinen in einer Menge von 0,001 bis 5 mol-%, bevorzugt von 0,005 bis 1 mol-%, bezogen auf die Verbindung der Formel (II), verwendet.

Es kann von Vorteil sein, das erfindungsgemäße Verfahren in Gegenwart von Basen durchzuführen. Geeignete Basen sind z.B. offenkettige oder cyclische sekundäre oder tertiäre Amine, wie z.B. Diethylamin, Triethylamin, Pyrrolidin, Piperidin, Piperazin, Diazabicyclooctan (DABCO), Diazabicyclononen (DBN), Diazabicycloundecen (DBU), Arylamine, Aryldiamine, Alkali- und Erdalkalisalze von aliphatischen und aromatischen Carbonsäuren, wie z.B. Natrium-, Kalium-oder Calciumacetat, Natrium- oder Kaliumpropionat, Natrium-oder Kaliumlaurat, Natrium- oder Kaliumbenzoat, Alkali- sowie Erdalkalicarbonate, wie z.B. Kaliumcarbonat, Natriumcarbonat, Lithiumcarbonat, Calciumcarbonat, Alkali- bzw. Erdalkalihydrogencarbonate, wie z.B. Natriumhydrogencarbonat, Calciumhydrogencarbonat oder auch Alkali- oder Erdalkalihydroxide, wie z.B. Lithiumhydroxid, Kaliumhydroxid, Natriumhydroxid, Calciumhydroxid, Bariumhydroxid. Die genannten Basen können alleine oder in beliebigen Mischungen untereinander eingesetzt werden.

Als Lösungsmittel geeignete Alkohole umfassen beispielsweise geradkettige oder verzweigte primäre, sekundäre oder tertiäre C₁-C₁₂-Alkohole oder Diole wie Methanol, Ethanol, Ethylenglykol, Propanol, Iso-Propanol, n-Butanol, tert.-Butanol, tert.-Amylalkohol, 2-Ethylhexanol.

Das erfindungsgemäße Verfahren wird im allgemeinen bei Temperaturen zwischen -20 und 150°C, vorzugsweise zwischen 0 und 120°C, besonders bevorzugt zwischen 20 und 90°C durchgeführt.

Insbesondere dient das erfindungsgemäße Verfahren zur Herstellung von Verbindungen der Formel (Ia) 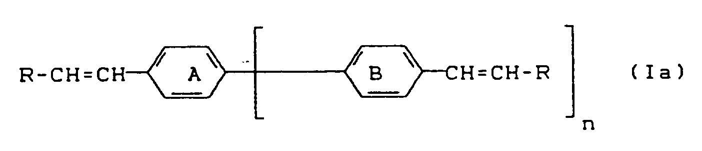
worin
- R und n: die für Formel (I) angegebene Bedeutung haben,
wobei für den Fall, daß n für Null steht, die freie Valenz am Ring A durch Wasserstoff oder einen für den Ring A genannten Substituenten abgesättigt ist, und die Ringe A und B weitere, wie bei Formel (I) für diese Ringe angegebene Substituenten tragen können,
durch Umsetzung einer Verbindung der Formel (IIa) 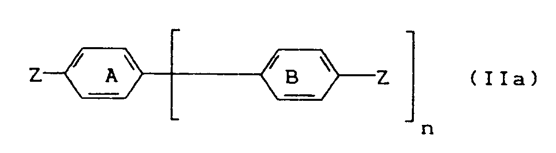
worin
- Z und n: die für Formel (II) angegebene Bedeutung haben,
und die Ringe A und B weitere, wie bei Formel (II) für diese Ringe angegebene Substituenten tragen können,
mit einer Verbindung der Formel (III).

In einer ebenfalls bevorzugten Ausführungsform des erfindungsgemäßen Verfahrens werden Verbindungen der Formel (II), vorzugsweise der Formel (IIb) 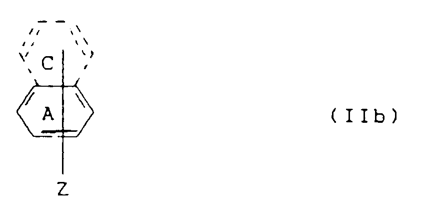
worin
- Z: die für Formel (II) angegebene Bedeutung hat,
und die Ringe A und C gegebenenfalls weitere, wie bei Formel (I) für diese Ringe definierte Reste tragen,
mit Ethylen (IIIa) umgesetzt.

Auf diese Weise werden bevorzugt Verbindungen der Formeln 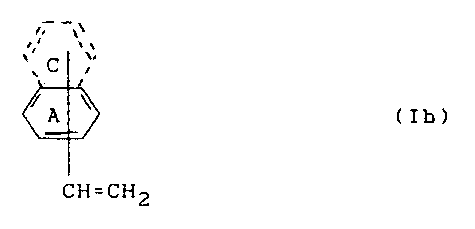
und 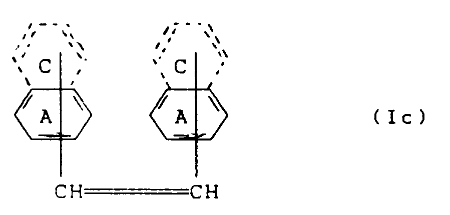
wobei die Ringe A und C jeweils weitere, wie unter Formel (I) für diese Ringe angegebene, Substituenten tragen können,
hergestellt.

Dabei läßt sich das Verhältnis von Vinylaromaten (Ib) zu Diarylethylenverbindungen (Ic) durch die Wahl der Reaktionsbedingungen, insbesondere auch durch die Art der Ethyleneinleitung, beeinflussen.

Im allgemeinen erhält man bevorzugt Vinylaromaten (Ib), wenn man die Reaktion unter einem Ethylendruck von 6 bis 100 bar, bevorzugt von 8 bis 50 bar durchführt. Nachteilig ist, daß man dabei auf ein Arbeiten in Autoklaven angewiesen ist.

Diese Reaktion kann aber auch bei niedrigeren als den oben genannten Drucken durchgeführt werden, wenn man das Ethylen mit Hilfe einer Intensivbegasungseinrichtung in die Reaktionsmischung einleitet.

Vorzugsweise wird die Umsetzung mit Ethylen bei Normaldruck oder leicht erhöhtem Druck durchgeführt, wobei das Ethylen mit Hilfe einer Intensivbegasungseinrichtung in die Reaktionsmischung eingeleitet wird.

Geeignete Intensivbegasungseinrichtungen sind z.B.: Oberflächenbegaser oder Volumenbegaser wie Kreiselbelüfter, Walzenbelüfter, Wasserstrahlbelüfter, Tauchstrahlbelüfter, Rohrrührer, Rührer mit separater Gaszufuhr, Tauchbelüfter, Fritten, Lochboden, statischer Mischer oder eine Zweistoffdüse, durch die das Reaktionsgemisch und das Ethylen simultan geführt werden.

Es kann günstig sein, die Intensivbegasungseinrichtung durch ein zusätzliches Mischorgan zur Homogenisierung der Reaktionsmischung zu ergänzen.

Solche Intensivbegasungseinrichtungen und Mischorgane sind dem Fachmann bekannt, beispielsweise aus Ullmann's Encyclopedia of Industrial Chemistry, 5. Aufl., Vol, B2. VCH Verlagsgesellschaft, Weinheim (1988).

Die Verfahrensprodukte der Formel (I) sind zum Teil bekannt.

Insbesondere dient das erfindungsgemäße Verfahren zur Herstellung von Verbindungen der Formel (VI) 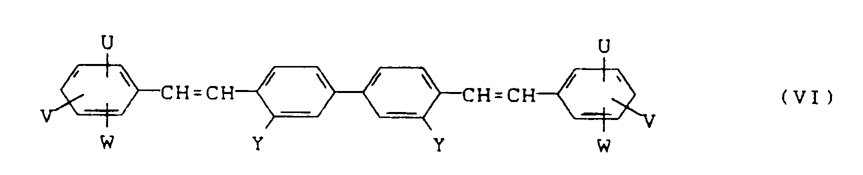
worin
- Y: für H oder SO₃M steht,
- U, V, W: unabhängig voneinander für H, F, Cl, Methyl, Methoxy, CF₃, CN, OCF₃, SO₃M, COOM, COO-C₁-C₁₂-Alkyl stehen,
und M die oben angegebene Bedeutung hat,
der Formel (VII) 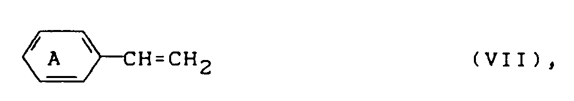
worin
der Ring A einen oder mehrere der oben unter Formel (I) für diesen Ring genannten Substituenten tragen kann,
sowie der Formeln (VIII) und (VIIIa) 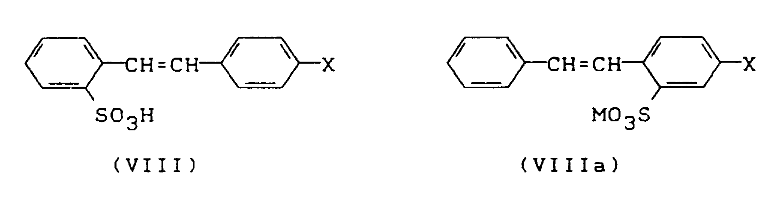
worin
- X: Chlor, Brom oder Iod bedeutet und
- M: die oben angegebene Bedeutung hat.

Die Verbindungen der Formeln (VI), (VII), (VIII) und (VIIIa) sind z.T. bekannte optische Aufheller bzw. Vorprodukte für optische Aufheller.

Bisher ging man bevorzugt so vor, daß man anstelle von Verbindungen der Formel (II) die entsprechenden Halogenverbindungen wie z.B. Brombenzol-2-sulfonsäure einsetzte (DE-A 23 25 302). Von Stellungsisomeren freie Halogenbenzolsulfonsäuren, wie die genannte Brombenzol-2-sulfonsäure, sind aber in der Regel schwerer zugänglich als die Verbindungen der Formel (II), sie werden sogar bevorzugt aus Verbindungen der Formel (II) durch Sandmeyer-Reaktion hergestellt. Das erfindungsgemäße Verfahren erlaubt es, die Stufe der Halogenbenzolsulfonsäuren einzusparen.

Weitere bevorzugte Verfahrensprodukte entsprechen der Formel (IX) 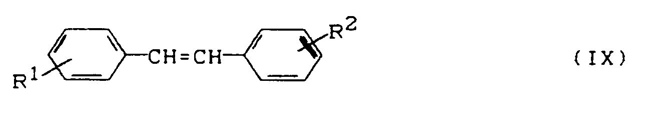
worin
R¹ und R² unabhängig voneinander für H, COOM, COO-C₁-C₁₂-Alkyl, NO₂, COO-C₆-C₁₀-Aryl, CO-C₁-C₁₂-Alkyl, CO-C₆-C₁₀-Aryl, O-C₆-C₁₂-Aryl, O-C₁-C₁₂-Alkyl stehen,
der Formel (X) 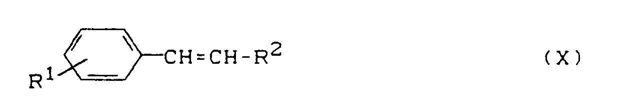
worin
R¹ und R² die obengenannte Bedeutung haben.

Als Beispiele für Verbindungen der Formel (IX) seien genannt:
Stilben-2-carbonsäure, Stilben-3-carbonsäure, Stilben-4-carbonsäure sowie die geradkettigen oder verzweigten C₁-C₁₂-Alkylester dieser Carbonsäuren, die sich z.B. von folgenden Alkohlen ableiten: Methanol, Ethanol, n-Propanol, i-Propanol, n-Butanol, i-Butanol, tert-Butanol, die isomeren Pentanole, Hexanole.

Ferner seien genannt: Stilben-4,4'-dicarbonsäure, Stilben-3,3'-dicarbonsäure, Stilben-2,2'-dicarbonsäure sowie die geradkettigen oder verzweigten C₁-C₁₂-Alkylester dieser Carbonsäuren, die sich ebenfalls von den obengenannten Alkoholen ableiten.

Stilben-2-carbonsäure bzw. Stilben-2-carbonsäurealkylester sind wichtige und nach den erfindungsgemäßen Verfahren einfach und preiswert zugängliche Vorstufen für Dibenzosuberon, einem Zwischenprodukt für Pharmazeutika.

Als Beispiele für Verbindungen der Formel (X) seien insbesondere Verbindungen genannt, in denen R¹ für H oder C₁-C₁₂-Alkoxy und R² für COOH oder COO-C₁-C₁₂-Alkyl stehen, wie z.B. Zimtsäure oder substituierte Zimtsäureester. Genannt sei p-Methoxyzimtsäure-2-ethyl-hexylester (XI), ein wichtiger UV-Absorber 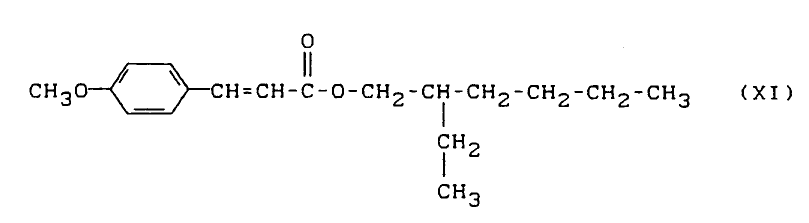
Die Verbindungen der Formel (II) können in an sich bekannter Weise aus Verbindungen der Formel (XII) 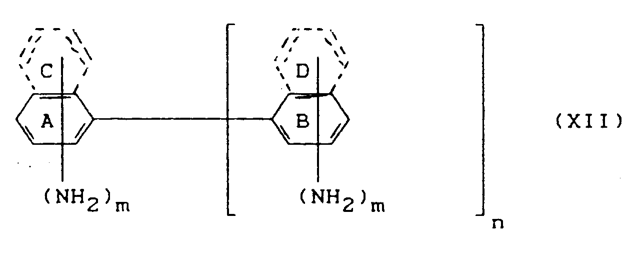
worin
- m und n: die oben genannte Bedeutung haben,
und die Ringe A, B, C und D gegebenenfalls weitere Substituenten, wie unter Formel (I) für diese Ringe aufgeführt, tragen,
beispielsweise durch Behandeln mit Nitritionen oder mit Alkylnitriten in Gegenwart von Säuren in wäßriger oder organischer Lösung hergestellt werden (vgl. z.B. Houben-Weyl, Methoden der Organischen Chemie, Band X/3, Georg Thieme Verlag, Stuttgart 1965, Seite 7 ff).

Beim Diazotieren in organischer Lösung kann es vorteilhaft sein, das entstehende Wasser z.B. durch wasserbindende Zusätze zu entfernen. Solche wasserbindenden Zusätze sind z.B. Natriumsulfat, Magnesiumsulfat, Kupfersulfat, Calciumchlorid oder auch Molekularsiebe.

In bestimmten Fällen ist es möglich, bei Anwesenheit mehrerer NH₂-Gruppen in den Verbindungen der Formel (XII) gezielte Diazotierungen vorzunehmen, siehe z.B. Houben-Weyl, Methoden der organischen Chemie, Georg Thieme Verlag, Stuttgart 1965, Band X/3, Seite 39 ff zur einfachen oder zweifachen Diazotierung aromatischer Diamine.

Die Diazoniumsalze der Formel (II) bzw. (IIa) und (IIb) lassen sich in bestimmten Fällen isolieren und in das erfindungsgemäße Verfahren einsetzen. Es ist aber auch möglich, die bei der Herstellung anfallende Lösung oder Suspension des Diazoniumsalzes einzusetzen. Man kann die Umsetzung in dieser Lösung oder Suspension des Diazoniumsalzes vornehmen, indem man mit der Verbindung der Formel (III) versetzt, gegebenenfalls Base, gegebenenfalls Komplexbildner, und Palladium-Katalysator zusetzt und den Reaktionsansatz, wenn nötig, bis zur Gasentwicklung erwärmt.

Man kann das erfindungsgemäße Verfahren aber auch so durchführen, daß zunächst in einem geeigneten Lösungsmittel Palladium-Katalysator, gegebenenfalls ein Komplexbildner, gegebenenfalls eine Base und die Verbindung der Formel (III) vorgelegt werden, und dann bei der zur Reaktion nötigen Temperatur das Diazoniumsalz der Formel (II) bzw. (IIa) oder (IIb) oder die Lösung bzw. Suspension des entsprechenden Diazoniumsalzes z.B. aus einem gegebenenfalls gekühlten Vorratsbehälter zudosiert werden.

Besonders dann, wenn die Verbindung der Formel (III) bei der Reaktionstemperatur flüssig ist, kann bei dieser Verfahrensvariante auf ein Lösungsmittel verzichtet werden.

Der Pd-Katalysator kann nach beendeter Reaktion zurückgewonnen werden, wobei die Art und Weise der Rückgewinnung sich unter anderem nach der Art des eingesetzten Katalysators und des verwendeten Lösungsmittels richtet. Im Prinzip wird eine Verteilung von Reaktionsprodukt und Katalysator auf verschiedene, gut voneinander trennbare Phasen angestrebt. Ist z.B. das Reaktionsprodukt im Reaktionsmedium löslich, der Katalysator aber nicht, sei es, weil er sich auf einem Träger wie z.B. Aktivkohle befindet oder weil er als metallisches Palladium vorliegt, dann läßt sich der Katalysator durch einfache Filtration abtrennen und das Reaktionsprodukt kann aus dem Filtrat isoliert werden. Liegt umgekehrt ein im Reaktionsgemisch löslicher Katalysator, z.B. ein Pd-Komplex, neben ungelöstem Produkt vor, kann zunächst das Produkt durch Filtration und anschließend der Katalysator aus dem Filtrat isoliert werden. Gegebenenfalls kann auch das den Katalysator enthaltende Filtrat erneut in das erfindungsgemäße Verfahren zurückgeführt werden. Liegen sowohl der Katalysator als auch das Reaktionsprodukt gelöst vor, kann man die Trennung durch geeignete Maßnahmen wie z.B. Aussalzen des Produkts, Ausfällen einer Komponente durch Zugabe eines Lösungsmittels geeigneter Polarität oder Extraktion einer Komponente mit einem geeigneten Lösungsmittel herbeiführen. Wenn Katalysator und Reaktionsprodukt in Reaktionsmedium unlöslich sind, lassen sich beide durch Filtration gemeinsam isolieren und anschließend z.B. durch Behandeln mit einem Lösungsmittel, das nur eine Komponente löst, trennen.

Der Katalysator kann, gegebenenfalls nach entsprechender Aufbereitung, wieder in das Verfahren zurückgeführt werden.

### Beispiele

### Beispiel 1

Zur Lösung von 5,2 g Anilin-2-sulfonsäure und 1,3 g NaOH in 40 ml H₂O gab man 13 ml konz. HCl und tropfte bei 0 bis 5°C 2,3 g NaNO₂ gelöst in 5 ml Wasser zu. Nach ca. 10 min wurde mit verdünnter NaOH neutralisiert. Man gab 3,46 g Na₂CO₃ zu, leitete über einen Rohrrührer Ethylen ein, gab 0,068 g Pd(OAc)₂ zu und rührte 2 h bei Raumtemperatur. Dann wurde zur Trockne eingedampft und das Produkt mit Methanol aus dem festen Rückstand extrahiert. Nach dem Eindampfen des Methanols verblieben 5,3 g (86 %) Styrol-2-sulfonsäure, Na-Salz.
- ¹H-NMR (DMSO-d₆):: 5,2 (dd, 1H)
5,7 (dd, 1H)
7,15-7,4 (m, 2H)
7,55-7,85 (m, 3H)

### Beispiel 2

Zu 17,3 g Anilin-2-sulfonsäure in 300 ml Methanol tropfte man zunächst 18 g Eisessig und dann 11,7 g Isoamylnitrit zu und ließ eine halbe Stunde bei Raumtemperatur nachrühren. Über einen Rohrrührer wurde Ethylen eingeleitet, man gab zunächst 113 mg Pd(OAc)₂ zu und dann portionsweise 27,6 g Kaliumcarbonat. Nach beendeter Zugabe wurde noch 1 Std. nachgerührt, dann ein Teil des Methanols abdestilliert. Man filtrierte von unlöslichen Salzen ab und dampfte das Filtrat zur Trockne ein.
Ausbeute: 18,4 g (83 %) Styrol-2-sulfonsäure, K-Salz.

### Beispiel 3

Es wurde verfahren wie in Beispiel 2, anstelle von Kaliumcarbonat wurde Natriumcarbonat verwendet.
Ausbeute: 16,3 g (79 %) Styrol-2-sulfonsäure, Na-Salz.

### Beispiel 4

Es wurde verfahren wie in Beispiel 2, anstelle von Kaliumcarbonat wurde eine Lösung von 16 g NaOH in 200 ml Methanol langsam zugegeben.
Ausbeute: 17,9 g (87 %) Styrol-2-sulfonsäure, Na-Salz,

### Beispiel 5

Zu 6.88 g 4,4'-Diaminobiphenyl-3,3'-disulfonsäure und 6,9 ml Eisessig in 100 ml Methanol tropfte man zwischen 10 und 20°C 5.32 ml Isoamylnitrit. Dann gab man 5,2 g Styrol und 45 mg Pd(OAc)₂ zu und tropfte eine Lösung von 6,4 g NaOH in 100 ml Methanol zu. Man rührte 2 Std. bei Raumtemperatur nach, engte zur Trockne ein, nahm den Rückstand in Wasser auf, filtrierte und salzte aus.
Ausbeute: 8,7 g (79 %) 4,4'-Distyrylbiphenyl-3,3'-disulfonsäure, Di-Na-Salz.
- ¹H-NMR-Spektrum: =: 7.21 (d, 2H), 7.30 (m, 2H), 7.41 (m, 4H), 7.57 (m, 4H), 7.68 (m, 2H), 7.92 (m, 2H), 8.18 (d, 2H), 8.33 (d, 2H).

### Beispiel 6

Zur 5,7 g 2-Aminobenzolsulfonsäure und 13 ml konz. HCl in 40 ml Wasser tropfte man bei 0 bis 5°C eine Lösung von 2,3 g NaNO₂ in 5 ml Wasser. Der Niederschlag wurde abgesaugt und zusammen mit 3,45 g Natriumcarbonat in 100 ml Wasser vorgelegt. Unter einem Druck von 50 bar Ethylen wurde bei Raumtemperatur eine Lösung von 68 mg Pd(OAc)₂ und 560 mg einer Verbindung der Formel 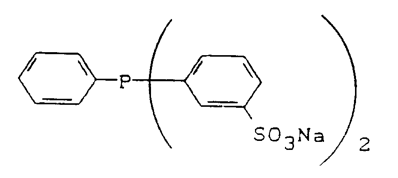
in 10 ml Wasser zugegeben und die Reaktionsmischung 2 Stunden bei Raumtemperatur gerührt. Dann wurde zur Trockne eingeengt und das Produkt mit Methanol aus dem Rückstand extrahiert. Man erhielt 4,8 g (78 %) Styrol-2-sulfonsäure, Na-Salz.

### Beispiel 7

Das Diazoniumsalz der 2-Aminobenzolsulfonsäure wurde wie in Beispiel 6 hergestellt, die entstandene Suspension wurde mit wäßriger Natronlauge neutralisiert und dann mit 3,46 g Natriumcarbonat versetzt. Unter einem Druck von 50 bar Ethylen wurde bei Raumtemperatur eine Lösung von 68 mg Pd(OAc)₂ und 560 mg einer Verbindung der Formel 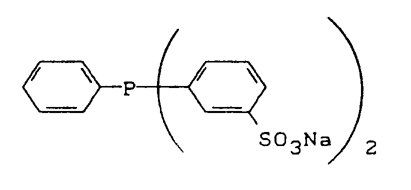
in 10 ml Wasser zugegeben und die Reaktionsmischung 2 Stunden bei Raumtemperatur gerührt. Nach Aufarbeitung wie in Beispiel 6 beschrieben, erhielt man 4,4 g (71 %) Styrol-2-sulfonsäure, Na-Salz.

### Beispiel 8

Es wurde verfahren wie in Beispiel 7, anstelle der Verbindung der Formel 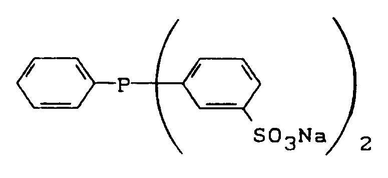
wurden jedoch 430 mg der Verbindung der Formel 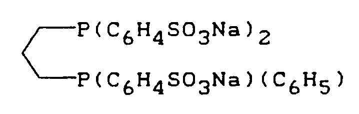
zugesetzt. Ausbeute: 4,7 g (77 %) Styrol-2-sulfonsäure, Na-Salz.

### Beispiel 9

In eine Suspension von 17,3 g Anilin-2-sulfonsäure in 300 ml Methanol wurden bei 40°C 10 g HCl-Gas eingeleitet. Der Ansatz wurde 2 Stunden bei dieser Temperatur nachgerührt, anschließend auf 20°C abgekühlt und tropfenweise mit 12,6 g Amylnitrit versetzt. Nach einer weiteren Stunde wurden 34 g Natriumacetat zugegeben und über einen Begasungsrührer 20 g Ethylen in die Suspension eingeleitet. Anschließend gab man 0,7 g Bis(benzonitril)palladium(II)-chlorid hinzu und rührte den Ansatz 5 Stunden unter einem lebhaften Ethylenstrom. Nach dieser Zeit wurde abgesaugt und das Filtrat zur Trockne eingedampft. Es verblieben 15,8 g (77 %) Styrol-2-sulfonsäure, Na-Salz.

### Beispiel 10

Man wiederholte das Beispiel 9, verwendete aber anstelle von Anilin-2-sulfonsäure die gleiche Menge Anilin-4-sulfonsäure und anstelle von Methanol das gleiche Volumen iso-Propanol. Es wurden 15,0 g (73 %) Styrol-4-sulfonsäure, Na-Salz erhalten.

### Beispiel 11

Zu 41,1 g Anthranilsäure und 24 ml konz. H₂SO₄ in 300 ml Wasser tropft man bei 0-5°C 20,7 g Natriumnitrit in 50 ml Wasser und läßt 20 Minuten bei dieser Temperatur nachrühren. Ein eventuell vorhandener Nitritüberschuß wird durch Zugabe von Amidosulfonsäure zerstört. Die Lösung des so bereiteten Diazoniumsalzes tropft man bei ca, 60°C zu einem schnell gerührten Gemisch von 62,4 g Styrol und 20 ml Wasser, gleich nach Beginn der Zugabe versetzt man mit 68 mg Pd(OAc)₂. Nach beendeter Zugabe und Gasentwicklung läßt man abkühlen, saugt ab, wäscht mit warmem Wasser nach und trocknet bei 60°C. Man erhält 62,5 g (93 %) Stilben-2-carbonsäure vom Fp. 158-159°C, Lit 158-160°C, Chem. Ber. 27, 2506 (1894).
- ¹H-NMR (CDCl₃): δ =: 7,0 (d, 1H) 7,2-7,4 (m, 4H), 7,43-7,60 (m, 3H), 7,7 (m, 1H); 7,95-8,15 (m, 2H), 10,2 (breit, 1H).

### Beispiel 12

Zu 30,2 g Anthranilsäuremethylester und 16 ml konz. H₂SO₄ in 200 ml Wasser tropft man bei 0-5°C eine Lösung von 13,8 g Natriumnitrit in 30 ml Wasser und rührt nach beendeter Zugabe noch 15 Minuten bei dieser Temperatur. Ein eventuell vorhandener Nitritüberschuß wird durch Zugabe von Amidosulfonsäure zerstört. Dann versetzt man mit 16,4 g Natriumacetat, 41,6 g Styrol und 45 mg Pd(OAc)₂ und erwärmt bis zum Ende der Gasentwicklung auf 60-70°C. Nach beendeter Gasentwicklung werden die Phasen getrennt, die wäßrige Phase noch 1 x mit Methylenchlorid ausgeschüttelt, die vereinigten organischen Phasen werden 2 x mit Wasser gewaschen, getrocknet und vom Lösungsmittel befreit, Man erhält 45,6 g (95 %) Stilben-2-carbonsäuremethylester, nach GC (SE 30, 100-300°C) 90 %ig.
- ¹H-NMR (CDCl₃): δ =: 3.9 (s, 3H), 6.98 (d, 1H), 7.2-7.6 (m, 7H), 7,7 (m, 1H), 7.85-8.05 (m, 2H)

### Beispiel 13

Man verfährt wie in Beispiel 12, setzt aber nur 22,5 mg Pd (OAc)₂ ein. Man erhält 44,3 g (93 %) Stilben-2-carbonsäuremethylester, nach GC (SE 30, 100-300°C) 84 %ig.

### Beispiel 14

Zur auf etwa 10°C gekühlten Lösung von 123 g p-Anisidin und 100 ml konz. H₂SO₄ in 400 ml Methanol gibt man langsam 117 g Amylnitrit und rührt nach beendeter Zugabe noch 40 Minuten ohne Kühlung. Man versetzt mit 95 g Acrylsäuremethylester und 112 mg Pd(OAc)₂ und erwärmt bis zum Ende der Gasentwicklung auf ca. 55°C. Nach dem Abkühlen wird abgesaugt, mit Wasser nachgewaschen und bei 40°C getrocknet. Man erhält 167 g (87 %) p-Methoxyzimtsäuremethylester, Fp. 88-89°C, Lit 89°C.

### Beispiel 15

Man verfährt wie in Beispiel 14, ersetzt aber Amylnitrit durch die entsprechende Menge Methylnitrit. Ausbeute 180 g (94 %).

### Beispiel 16

Zur auf etwa 10°C gekühlten Mischung von 123 g p-Anisidin und 100 ml konz. H₂SO₄ in 500 ml 2-Ethylhexanol gibt man langsam 117 g Amylnitrit und rührt nach beendeter Zugabe noch 40 min. Man versetzt mit 202 g Acrylsäure-(2-ethylhexylester) und 112 mg Pd(OAc)₂ und erwärmt bis zum Ende der Gasentwicklung auf ca. 65°C.

Man verdünnt mit Wasser und extrahiert das Produkt mit Methylenchlorid. Die organische Phase wird noch 2 mal mit Wasser gewaschen, getrocknet und eingedampft. Der Rückstand wird im Vakuum destilliert. Man erhält 241 g (83 %) p-Methoxyzimtsäure-(2-ethylhexylester).
- ¹H-NMR (CDCl₃): δ =: 0,8-1,9 (m, 15H), 3,8 (s, 3H) 4,1 (m, 2H), 6,3 (d, 1H), 6,9 (m, 2H), 7,5 (m, 2H), 7,7 (d, 1H)

## Patentansprüche

1. Verfahren zur Herstellung von Verbindungen der Formel (I) 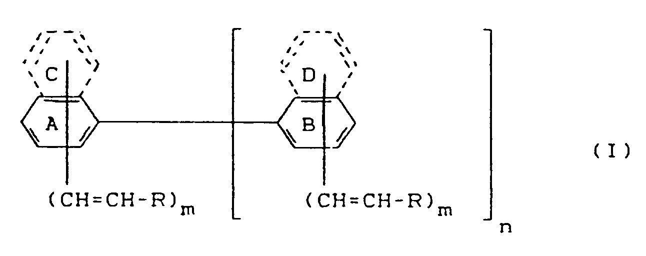 worin
R für H, CN, COOM, COO-C₁-C₁₂-Alkyl, SO₃M C₁-C₁₂-Alkyl, gegebenenfalls substituiertes C₆-C₁₀-Aryl, gegebenenfalls substituiertes Hetaryl mit 3 bis 5 Kohlenstoffatomen und 1 bis 3 Heteroatomen aus der Reihe Sauerstoff, Schwefel, Stickstoff, steht,
n für 0 oder 1 steht,
wobei für den Fall, daß n für Null steht, die freie Valenz an Ring A durch Wasserstoff abgesättigt ist,
m für 1 oder 2 steht
und
die Ringe A, B, C, D und die genannten Aryl- oder Hetarylreste weiterhin durch einen oder mehrere Substituenten aus der Reihe Fluor, Chlor, Brom, Iod, C₁-C₁₂-Alkyl, C₁-C₁₂-Alkoxy, CF₃, OCF₃, CN, NO₂, COOM, COO-C₁-C₁₂-Alkyl, SO₃M, CO-C₁-C₁₂-Alkyl, CO-C₆-C₁₀-Aryl, C₆-C₁₀-Aryl, Hetaryl, gegebenenfalls durch C₁-C₁₂-Alkyl substituiertes Amino, substituiert sein können und
M ein Proton, ein Alkalimetallion, ein Erdalkalimetallion oder Ammonium bedeutet
durch Umsetzung einer Verbindung der Formel (II) 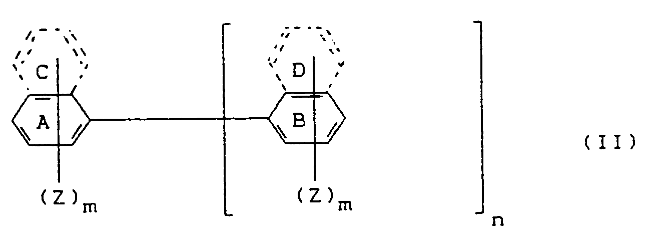 worin
Z für N₂^{⊕}G steht,
G für Sulfat oder Hydrogensulfat steht und
m und n die oben genannte Bedeutung haben,
wobei für den Fall, daß n für Null steht, die freie Valenz an Ring A durch Wasserstoff abgesättigt ist,
und die Ringe A, B, C und D gegebenenfalls durch einen oder mehrere der oben für diese Ringe genannten Substituenten substituiert sein können
mit einer Verbindung der Formel (III)
(III) R-CH=CH₂
worin
R die oben genannte Bedeutung hat in Gegenwart eines anorganischen oder organischen Palladiumsalzes oder deren Mischung als Katalysator, wobei die Reaktion in Wasser, Alkohol oder einer Mischung daraus durchgeführt wird.

2. Verfahren nach Ansprüche 1, dadurch gekennzeichnet, daß man die Umsetzung in Gegenwart einer Base durchführt.

3. Verfahren nach Ansprüchen 1 und 2 zur Herstellung von Verbindungen der Formel (Ia) 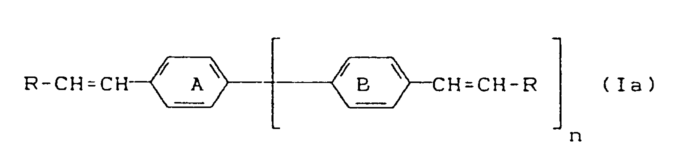 worin
R und n die in Anspruch 1 angegebene Bedeutung haben,
wobei für den Fall, daß n für Null steht, die freie Valenz am Ring A durch Wasserstoff oder einen für den Ring A genannten Substituenten abgesättigt ist, und die Ringe A und B weitere, wie in Anspruch 1 für diese Ringe angegebene Substituenten tragen können,
durch Umsetzung einer Verbindung der Formel (IIa) 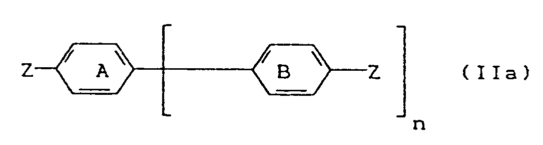 worin
Z und n die in Anspruch 1 angegebene Bedeutung haben, und die Ringe A und B weitere, wie in Anspruch 1 für diese Ringe angegebene Substituenten tragen können
mit einer Verbindung der Formel (III)
R-CH=CH₂ (III)
worin
R die in Anspruch 1 angegebene Bedeutung hat.

4. Verfahren nach Ansprüchen 1 bis 3, dadurch gekennzeichnet, daß Verbindungen der Formel (II) bzw. (IIa) mit Ethylen umgesetzt werden.

5. Verfahren nach Ansprüchen 1 bis 4, dadurch gekennzeichnet, daß als Aryldiazoniumsalze der Formel (II) bzw. (IIa) Sulfoaryldiazoniumsalze eingesetzt werden.

6. Verfahren nach Ansprüchen 1 bis 5, dadurch gekennzeichnet, daß der Rest R in der Formel (III) für H oder gegebenenfalls substituiertes Phenyl steht.

7. Verfahren nach Ansprüchen 1 bis 6, dadurch gekennzeichnet, daß als Aryldiazoniumsalze der Formel (II) bzw. (IIa) Sulfobenzoldiazoniumsalze eingesetzt werden.

8. Verfahren nach Ansprüche 4 bis 7, dadurch gekennzeichnet, daß das Ethylen mit Hilfe einer Intensivbegasungseinrichtung in das Reaktionsgemisch eingeleitet wird.

9. Verfahren nach Ansprüchen 4 bis 8, dadurch gekennzeichnet, daß als Intensivbegasungseinrichtung für das Ethylen ein Oberflächenbegaser oder ein Volumenbegaser, bevorzugt ein Volumenbegaser eingesetzt wird, wobei die Reaktionsmischung gegebenenfalls durch ein zusätzliches Mischorgan homogenisiert wird.

## Claims

1. Process for preparing compounds of the formula (I) 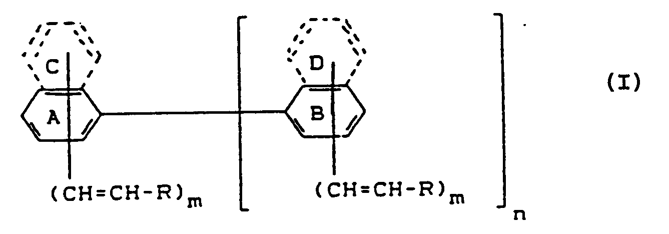 in which
R represents H, CN, COOM, COO-C₁-C₁₂-alkyl, SO₃M, C₁-C₁₂-alkyl, optionally substituted C₆-C₁₀-aryl, optionally substituted hetaryl having 3 to 5 carbon atoms and 1 to 3 heteroatoms from the series comprising oxygen, sulphur and nitrogen, and also represents
n represents 0 or 1,
where in the case in which n represents zero, the free valency on ring A is saturated by hydrogen,
m represents 1 or 2
and
the rings A, B, C, D and the aryl or hetaryl radicals mentioned can furthermore be substituted by one or more substituents from the series comprising fluorine, chlorine, bromine, iodine, C₁-C₁₂-alkyl, C₁-C₁₂-alkoxy, CF₃, OCF₃, CN, NO₂, COOM, COO-C₁-C₁₂-alkyl, SO₃M, CO-C₁-C₁₂-alkyl, CO-C₆-C₁₀-aryl, C₆-C₁₀-aryl, hetaryl, amino optionally substituted by C₁-C₁₂-alkyl, and
M denotes a proton, an alkali metal ion, an alkaline earth metal ion or ammonium
by reaction of a compound of the formula (II) 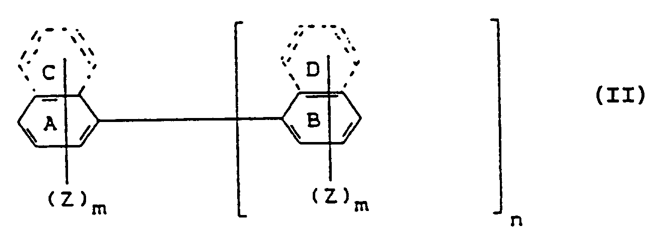 in which
Z represents N₂^{⊕}G,
G represents a sulphate or hydrogen sulphate and
m and n have the abovementioned meaning,
where in the case in which n represents zero, the free valency on ring A is saturated by hydrogen,
and the rings A, B, C and D can optionally be substituted by one or more of the substituents mentioned above for these rings,
with a compound of the formula (III)
(III) R-CH=CH₂
in which
R has the abovementioned meaning in the presence of an inorganic or organic palladium salt or a mixture thereof as catalyst, the reaction being carried out in water, alcohol or a mixture thereof.

2. Process according to Claim 1, characterised in that the reaction is carried out in the presence of a base.

3. Process according to Claims 1 and 2 for preparing compounds of the formula (Ia) 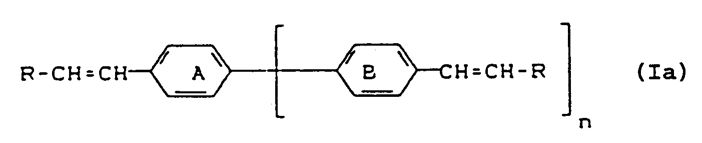 in which
R and n have the meaning indicated in Claim 1,
where, in the case in which n represents zero, the free valency on the ring A is saturated by hydrogen or a substituent mentioned for the ring A, and the rings A and B can carry further substituents, as indicated in Claim 1 for these rings,
by reaction of a compound of the formula (IIa) 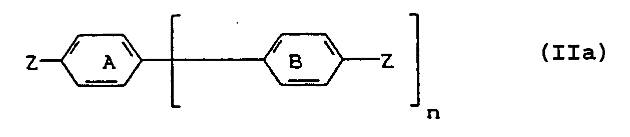 in which
Z and n have the meaning indicated in Claim 1, and the rings A and B can carry further substituents, as indicated in Claim 1 for these rings, with a compound of the formula (III)
R-CH=CH₂ (III)
in which
R has the meaning indicated in Claim 1.

4. Process according to Claims 1 to 3, characterised in that compounds of the formula (II) or (IIa) are reacted with ethylene.

5. Process according to Claims 1 to 4, characterised in that, as aryldiazonium salts of the formula (II) or (IIa), sulphoaryldiazonium salts are employed.

6. Process according to Claims 1 to 5, characterised in that the radical R in the formula (III) represents H or optionally substituted phenyl.

7. Process according to Claims 1 to 6, characterised in that, as aryldiazonium salts of the formula (II) or (IIa), sulphobenzenediazonium salts are employed.

8. Process according to Claims 4 to 7, characterised in that the ethylene is passed into the reaction mixture with the aid of an intensive aeration device.

9. Process according to Claims 4 to 8 characterised in that the intensive aeration device for the ethylene is a surface aerator or a volume aerator, preferably a volume aerator, the reaction mixture optionally being homogenised by an additional mixing element.

## Revendications

1. Procédé de préparation de composés de formule (I) 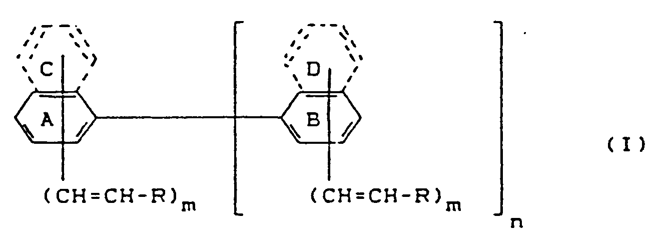 dans laquelle
R représente H, CN, COOM, COO-(alkyle en C₁-C₁₂), SO₃M, alkyle en C₁-C₁₂, aryle en C₆-C₁₀ éventuellement substitué, hétéroaryle éventuellement substitué ayant 3 à 5 atomes de carbone et 1 à 3 hétéroatomes de la série de l'oxygène, du soufre, de l'azote,
n représente 0 ou 1, dans laquelle, lorsque n est nul, la valence libre du cycle A est saturée par l'hydrogène,
m représente 1 ou 2,
et
les cycles A, B, C, D et les restes aryle ou hétéroaryle cités peuvent être substitués en outre par un ou plusieurs substituants de la série suivante : fluor, chlore, brome, iode, alkyle en C₁-C₁₂, alcoxy en C₁-C₁₂, CF₃, OCF₃, CN, NO₂, COOM, COO-(alkyle en C₁-C₁₂), SO₃M, CO-(alkyle en C₁-C₁₂), CO-(aryle en C₆-C₁₀), aryle en C₆-C₁₀, hétéroaryle, amino éventuellement substitué par alkyle en C₁-C₁₂, et
M représente un proton, un ion de métal alcalin, un ion de métal alcalino-terreux ou un ammonium
par réaction d'un composé de formule (II) 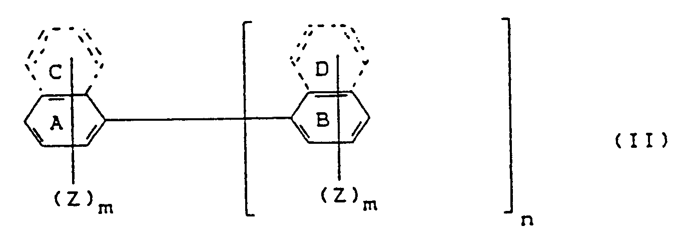 dans laquelle
Z représente N₂^{⊕}G,
G représente un sulfate ou hydrogénosulfate et
m et n ont la signification citée ci-dessus,
dans laquelle, lorsque n est nul, la valence libre du cycle A est saturée par l'hydrogène,
et les cycles A, B, C et D peuvent éventuellement être substitués par un ou plusieurs des substituants cités ci-dessus pour ces cycles,
avec un composé de formule (III)
R-CH=CH₂ (III)
dans laquelle
R a la signification citée,
en présence d'un sel de palladium inorganique ou organique ou d'un mélange de tels sels en tant que catalyseur, la réaction étant conduite dans l'eau, dans un alcool ou dans un mélange de ceux-ci.

2. Procédé selon la revendication 1, caractérisé en ce que l'on conduit la réaction en présence d'une base.

3. Procédé selon les revendications 1 et 2 de préparation de composés de formule (Ia) 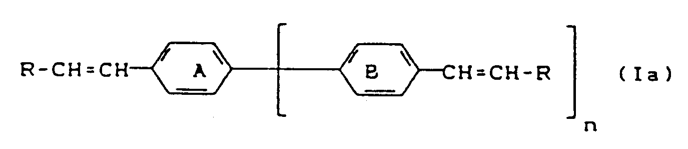 dans laquelle
R et n ont la signification indiquée dans la revendication 1,
dans laquelle, lorsque n est nul, la valence libre du cycle A est saturée par l'hydrogène ou un substituant cité pour le cycle A, et les cycles A et B peuvent porter d'autres substituants tels que ceux indiqués pour ces cycles dans la revendication 1,
par réaction d'un composé de formule (IIa) 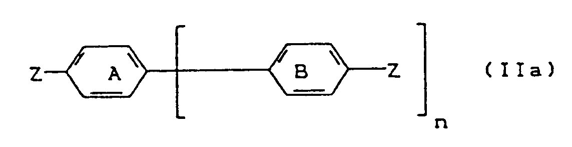 dans laquelle
Z et n ont la signification indiquée dans la revendication 1, et les cycles A et B peuvent porter d'autres substituants tels que ceux indiqués dans la revendication 1 pour ces cycles
avec un composé de formule (III)
R-CH=CH₂ (III)
dans laquelle
R a la signification indiquée dans la revendication 1.

4. Procédé selon les revendications 1 à 3, caractérisé en ce que des composés de formule (II) ou (IIa) sont mis à réagir avec l'éthylène.

5. Procédé selon les revendications 1 à 4, caractérisé en ce que des sels de sulfoaryldiazonium sont utilisés comme sels d'aryldiazonium de formule (II) ou (IIa).

6. Procédé selon les revendications 1 à 5, caractérisé en ce que le reste R dans la formule (III) représente H ou un phényle éventuellement substitué.

7. Procédé selon les revendications 1 à 6, caractérisé en ce que des sels de sulfobenzènedizonium sont utilisés comme sels d'aryldiazonium de formule (II) ou (IIa).

8. Procédé selon les revendications 4 à 7, caractérisé en ce que l'éthylène est introduit dans le mélange réactionnel au moyen d'un dispositif d'introduction intensive de gaz.

9. Procédé selon les revendications 4 à 8, caractérisé en ce que l'on utilise comme dispositif d'introduction intensive de gaz pour l'éthylène un dispositif d'introduction de gaz superficiel ou un dispositif d'introduction de gaz volumique, de préférence un dispositif d'introduction de gaz volumique, le mélange réactionnel étant éventuellement homogénéisé par un organe mélangeur supplémentaire.
